(19) Europäisches Patentamt
European Patent Office
Office européen des brevets

(11) Veröffentlichungsnummer: **0 002 508**
**B1**

(12) # EUROPÄISCHE PATENTSCHRIFT

(45) Veröffentlichungstag der Patentschrift: **31.03.82**

(51) Int. Cl.³: **C 07 D 313/14**, C 07 D 407/12, A 61 K 31/335

(21) Anmeldenummer: **78101618.3**

(22) Anmeldetag: **08.12.78**

(54) **Aminoalkylthiodibenzoxepine, Verfahren zu deren Darstellung sowie diese enthaltende Arzneimittel.**

(30) Priorität: **13.12.77 US 860083**

(43) Veröffentlichungstag der Anmeldung: **27.06.79 Patentblatt 79/13**

(45) Bekanntmachung des Hinweises auf die Patenterteilung: **31.03.82 Patentblatt 82/13**

(84) Benannte Vertragsstaaten: **BE CH DE FR GB IT NL SE**

(56) Entgegenhaltungen: **CH-A-509 306**

(73) Patentinhaber: **HOECHST AKTIENGESELLSCHAFT, Postfach 80 03 20, D-6230 Frankfurt/Main 80 (DE)**

(72) Erfinder: **Ong, Helen Hu, Bunker Hill Place 15, Whippany N.J. (US)**
Erfinder: **Anderson, Vernon Brian, Sylvan Road 24, High Bridge N.J. (US)**
Erfinder: **Profitt, James Arthur, Robbins Road 62, Somerville N.J. (US)**

**EP 0 002 508 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Aminoalkylthiodibenzoxepine, Verfahren zu deren Darstellung sowie diese enthaltende Arzneimittel

Die vorliegende Erfindung betrifft neue Aminoalkylthiodibenzoxepine und ihre physiologisch unbedenklichen Säureadditionssalze, welche als Antidepressiva, Analgetika und Antikonvulsiva verwendet werden können, sowie Arzneimittel die eine solche Verbindung als Wirkstoff enthalten.

Es ist bereits bekannt, daß Amethoclothepin der Formel

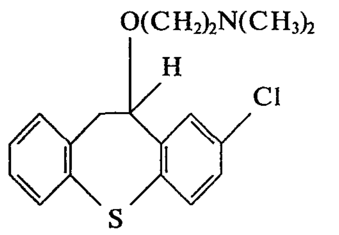

nach M. Protvia u. a., Il Farmaco XXI, 98 (1966), eine zentral-depressive Wirkung besitzt.

Gemäß der japanischen Patentschrift Nr. 47-28 998 mit dem Titel »Verfahren zur Herstellung tricyclischer Verbindungen mit einer Enolätherbindung« handelt es sich um die Herstellung von Verbindungen, welche durch die Formel

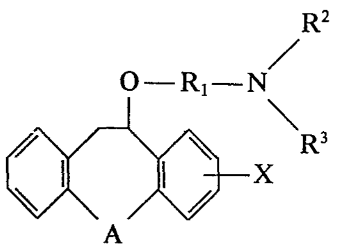

worin A Alkylimino, Oxy, Thio oder Sulfinyl und $R_1$ Alkylen bedeutet, $R_2$ und $R_3$ je eine Alkylgruppe darstellen oder gegebenenfalls über eine Alkyliminogruppe cyclisch gebunden sein können, und X für Wasserstoff, Halogen, Alkyl, Alkoxy, Alkylthio, Dialkylsulfamoyl oder Nitro steht, wiedergegeben werden.

Die vorliegende Erfindung betrifft Verbindungen der Formel

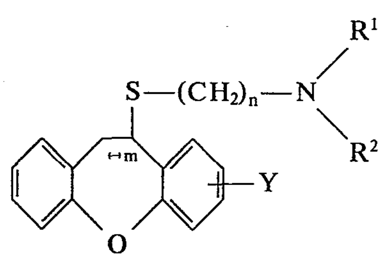

(I)

worin Y für Wasserstoff, Halogen, $C_1-C_4$-Alkyl, $C_1-C_6$-Alkylthio, $C_1-C_4$-Alkylsulfonyl, steht $R^1$ Wasserstoff, geradkettiges oder verzweigtes $C_1-C_4$-Alkyl, Cycloalkyl-$C_1-C_3$-alkyl, wobei der Cycloalkylring 3 bis 5 Kohlenstoffatome aufweist, oder Propargyl und $R^2$ geradkettiges oder verzweigtes $C_1-C_4$-Alkyl bedeutet, oder $R^1$ und $R^2$ zusammen mit dem Stickstoffatom, an das sie gebunden sind, einen Hetercyclus bilden, welcher Morpholin, Piperazinyl oder N-substituiertes Piperazinyl mit $C_{1-3}$-Alkyl als N-Substituent darstellt, m die Zahl 0 oder 1 bedeutet, und n für eine ganze Zahl von 2 bis 4 steht, sowie deren physiologisch unbedenkliche Säureadditionssalze.

Von den genannten Resten sind die folgenden bevorzugt:

Für Y: Wasserstoff, Fluor, Chlor, Brom, $C_1-C_4$-Alkyl, $C_1-C_4$-Alkylsulfonyl,
für $R^1$: Wasserstoff, $C_1-C_4$-Alkyl, $C_3-C_5$-Cycloalkyl-$C_1-C_3$-alkyl,
für $R^2$: $C_1-C_4$-Alkyl oder
für $R^1$ und $R^2$ zusammen mit dem N-Atom, an das sie gebunden sind, Morpholino, Piperazinyl oder N-substituiertes Piperazinyl mit $C_1-C_3$-Alkyl als N-Substituent.

Bei der Herstellung der erfindungsgemäßen physiologisch verträglichen Säureadditionssalze verwendbare Säuren sind unter anderem anorganische Säuren wie Salz-, Bromwasserstoff-,

Schwefel-, Salpeter-, Phosphor- und Perchlorsäure sowie organische Säuren wie Wein-, Zitronen-, Essig-, Bernstein-, Malein-, Fumar- und Oxalsäure.

Die erfindungsgemäßen Verbindungen stellt man nach den unten erläuterten Methoden her. Dabei haben Y, $R^1$, $R^2$, m und n die oben genannte Bedeutung, falls nicht anders angegeben.

### Methode a

Man setzt ein 10,11-Dihydro-10-hydroxy- oder -10-oxo-dibenz[b,f]oxepin der Formel

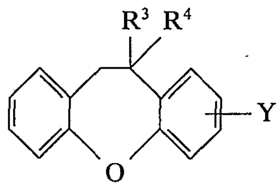

(II)

worin $R^3$ und $R^4$ verschieden sind und jeweils für Wasserstoff oder Hydroxyl oder zusammen für Sauerstoff stehen, mit einem Aminoalkylthio der Formel

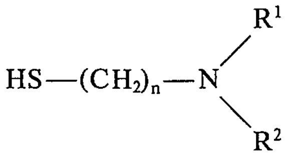

(III)

worin $R^1$ und $R^2$ gleich oder verschieden sind und jeweils für geradkettiges oder verzweigtes $C_2-C_4$-Alkyl stehen, zu einer Verbindung der Formel

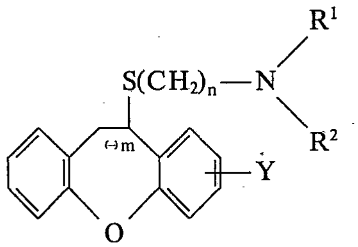

(I)

um. Diese Reaktion wird mit Borfluoridätherat als Katalysator/Dehydratisierungsmittel und in Gegenwart eines geeigneten Lösungsmittels wie Eisessig bei etwa Raumtemperatur bis Rückflußtemperatur durchgeführt.

### Methode b

Eine nach Methode a) hergestellte Verbindung, worin m die ganze Zahl 1 darstellt, kann man in einem geeigneten Lösungsmittel mit Magnesium behandeln, um eine Reduktion zum entsprechenden gesättigten Aminoalkylthiodibenzoxepin zu erzielen. Bei einer bevorzugten Methode zur Durchführung dieser Reduktion handelt es sich um die Verwendung von Magnesiumspänen mit Methanol als Lösungsmittel.

### Methoce c

Eine nach Methode a) oder b) hergestellte Verbindung, worin $R^1$ und $R^2$ je für Methyl stehen, kann in einem geeigneten Lösungsmittel sowie säurebindenden Mittel mit einem Halogencyan wie Bromcyan behandelt werden, wobei man ein Gemisch aus einer Verbindung der Formel

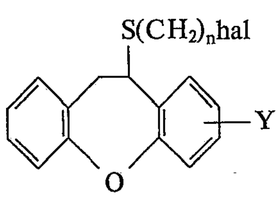

(IVa)

und einer Verbindung der Formel

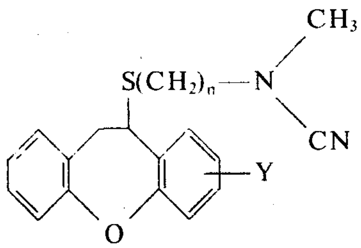

(IVb)

erhält. Diese Reaktion wird bei etwa Raumtemperatur bis Rückflußtemperatur durchgeführt. Die beiden Verbindungen können isoliert oder durch Säulenchromatographie gewonnen werden.

Methode d

Eine nach Methode c) hergestellte Verbindung der Formel IVa kann man in bekannter Weise mit einem geeigneten Amin umsetzen, wobei die entsprechende Verbindung der Formel

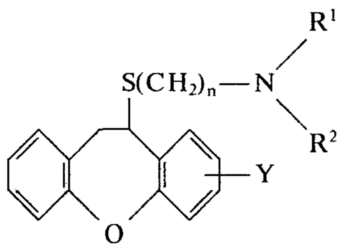

(I)

worin $R^1$ für Wasserstoff, geradkettiges oder verzweigtes $C_1-C_4$-Alkyl, Cycloalkyl-$C_1-C_3$-alkyl, oder Propargyl und $R^2$ für geradkettiges oder verzweigtes $C_1-C_4$-Alkyl oder Cycloalkyl-$C_1-C_3$-alkyl steht, $R^1$ und $R^2$ zusammen mit dem Stickstoffatom, an das sie gebunden sind, einen Heterocyclus bilden, welcher Piperazinyl, N-substituiertes Piperazinyl oder Morpholino darstellt. Eine bevorzugte Umsetzung nach dieser Methode wird mit Dimethylformamid als Lösungsmittel, einem Katalysator wie Natriumbicarbonat und einem Reaktionsinitiator wie Kaliumjodid bei Raumtemperatur bis zur Rückflußtemperatur des Reaktionsgemischs durchgeführt.

Methode e

Eine nach Methode a) oder b) hergestellte Verbindung kann man bei einer Temperatur von 25 bis 125°C, in einem Lösungsmittel wie Methylenchlorid, Toluol oder Benzol mit einem Chlorameisensäureester, z. B. Chlorameisensäurealkyl- oder -phenylester, behandeln, wobei die entsprechende erfindungsgemäße Verbindung, worin $R^1$ $C_1-C_6$-Alkoxy- oder Phenoxycarbonyl bedeutet, gebildet wird.

Methode f

Eine nach Methode e) hergestellte Verbindung wird in einem Lösungsmittel wie Wasser, Äthanol oder Äthylenglykol bei Raumtemperatur bis Rückflußtemperatur mit einer organischen Base wie Triäthylamin oder einer anorganischen Base wie Natrium- oder Kaliumhydroxid behandelt, wobei die entsprechende erfindungsgemäße Verbindung, worin $R^1$ für Wasserstoff steht, gebildet wird.

Methode g

Eine nach Methode f) hergestellte Verbindung wird unter den für solche Umsetzungen üblichen Bedingungen mit einem geradkettigen oder verzweigten $C_1-C_4$-Alkylhalogenid, Propargylhalogenid oder Cycloalkyl-$C_1-C_3$-alkylhalogenid behandelt, wobei die entsprechende erfindungsgemäße Verbindung, worin $R^1$ für geradkettiges oder verzweigtes $C_1-C_4$-Alkyl, Propargyl oder Cycloalkyl-$C_1-C_3$-alkyl steht, gebildet wird. Vorzugsweise erfolgt diese Substitution in Gegenwart eines Lösungsmittels wie Dimethylformamid, eines säurebindenden Mittels wie Natriumbicarbonat und eines Reaktionsinitiators wie Kaliumjodid bei der Rückflußtemperatur des Lösungsmittels.

Es ist für den Fachmann selbstverständlich, daß die speziellen Reaktionsbedingungen für die obigen Methoden von den eingesetzten Reaktionspartnern bei der jeweiligen Arbeitsweise abhängen.

Die erfindungsgemäßen Verbindungen sind zur Behandlung von Depressionen geeignet, wie ihre Fähigkeit an der Maus die durch Tetrabenazin induzierte Depression [International Journal of Neuropharmacology 8, 73 [1969]), ein Standardtest auf antidepressive Eigenschaften, zu hemmen, zeigt. So beträgt beispielsweise die intraperitoneale Dosis, bei der die folgenden Verbindungen eine 50%ige Hemmung der Ptosis (Oberlidlähmung) bei durch Tetrabenazin induzierter Depression ($ED_{50}$) an der Maus bewirken:

| Verbindung | $ED_{50}$ mg/kg |
|---|---|
| 2-Fluor-11-[β-(methylamino)-äthylthio]-dibenz[b,f]oxepin-maleinat | 0,3 |
| 10-[β-(Methylamino)-äthylthio]-dibenz[b,f]oxepin-oxalat | 3,4 |
| 2-Chlor-10,11-dihydro-11-[(β-dimethylamino)-äthylthio]-dibenz[b,f]oxepin-oxalat | 3,5 |
| 2-Fluor-11[β-(dimethylamino)-äthylthio]-dibenz[b,f]oxepin-hydrobromid | 4,3 |
| 10,11-Dihydro-10-[(β-dimethylamino)-äthylthio]-dibenz[b,f]oxepin-oxalat | 7,0 |
| 2-Fluor-10,11-dihydro-11-[(β-dimethylamino)-äthylthio]dibenz[b,f]oxepin-oxalat | 7,6*) |

*) perorale Dosis

Diese Werte zeigen, daß die erfindungsgemäßen Verbindungen bei Verabreichung in einer Tagesmenge von 0,1 bis 50 mg pro kg Körpergewicht, antidepressive Wirkung haben.

Erfindungsgemäße Verbindungen sind weiterhin wegen ihrer Fähigkeit, bei Säugetieren Schmerzen zu lindern, als Analgetika wertvoll. Die analgetische Wirkung der erfindungsgemäßen Verbindungen zeigt sich beim Phenyl-p-chinon-Streck-Test an der Maus, einem Standardtest für Analgetica (Proc. Soc. Exptl. Biol. Med., 95, 729 [1957]). So ist beispielsweise die subkutane Dosis, die eine ungefähr 50%ige Hemmung des bei diesem Test an der Maus hervorgerufenen Streckung ($ED_{50}$) bewirkt, wie folgt:

| Verbindung | $ED_{50}$ mg/kg |
|---|---|
| 2-Fluor-10,11-dihydro-11-[(β--dimethylamino)-äthylthio]dibenz[b,f]oxepin-oxalat | 1,9 |
| 2-Fluor-11-[β-(methylamino)-äthylthio]-dibenz[b,f]oxepin-maleinat | 2,3 |
| 10-[β-(Methylamino)-äthylthio]-dibenz[b,f]oxepin-oxalat | 2,3 |

Diese Werte zeigen, daß die erfindungsgemäßen Verbindungen zur Linderung von Schmerzen in Säugetieren bei Verabreichung in einer Tagesmenge von 0,1 bis etwa 50 mg pro kg Körpergewicht wirksam sind.

Verbindungen der Formel I sind weiterhin auch als Antikonvulsiva für Säugetiere wertvoll, wie nach der Methode von Woodbury, L. A. und Davenport, V. D. in Arch. Int. Pharmacodynam, Band 92, (1952) auf Seiten 97 bis 107, ermittelt. So sind beispielsweise die intraperitonealen Dosierungen für einen ungefähr 50%igen Schutz ($ED_{50}$) gegen die Auswirkung eines über dem Maximum liegenden Elektroschocks an der Maus wie folgt:

| Verbindung | $ED_{50}$ mg/kg |
|---|---|
| 10,11-Dihydro-10-[(β-dimethylamino)-äthylthio]-dibenz[b,f]oxepin-oxalat | 7,7 |
| 2-Fluor-10,11-dihydro-11-[(β-dimethylamino)-äthylthio]dibenz[b,f]oxepin-oxalat | 9,0 |
| 2-Fluor-11-[β-(dimethylamino)-äthylthio]-dibenz[b,f]oxepin-hydrobromid | 9,2 |
| 2-Fluor-11[β-(methylamino)-äthylthio]-dibenz[b,f]oxepin-maleinat | 9,9 |
| 2-Chlor-10,11-dihydro-11-[β-dimethylamino)-äthylthio]-dibenz[b,f]oxepin-oxalat | 19,4 |
| 10-[β-(Methylamino)-äthylthio]-dibenz[b,f]oxepin-oxalat | 21 |

Diese Werte zeigen, daß die erfindungsgemäßen Verbindungen zur Behandlung von Krämpfen in Säugetieren bei Verabreichung in einer Tagesmenge von etwa 0,1 bis 100 mg pro kg Körpergewicht wirksam sind.

Weitere Beispiele für erfindungsgemäße Verbindungen sind:

11-[γ-(Dimethylamino)-propylthio]-2-äthylsulfonyldibenz[b,f]oxepin;
2-Äthyl-11-[β-(methylamino)-äthylthio]-dibenz[b,f]oxepin;
11-[β-(Äthylmethylamino)-äthylthio]-2-methylsulfinyldibenz[b,f]oxepin;
10,11-Dihydro-10-[γ-(piperazinyl)-propylthio]-dibenz[b,f]oxepin;
3-Fluor-11-[β-(methylamino)-äthylthio]-dibenz[b,f]oxepin;
3-Äthyl-11-[β-(methylamino)-äthylthio]-dibenz[b,f]oxepin; und
2-Methyl-11-[β-(N-methyl-N-methoxycarbonyl)-aminoäthylthio]-dibenz[b,f]oxepin.

Wirksame Mengen der erfindungsgemäßen Verbindungen können einem Patienten nach verschiedenen Methoden verabreicht werden, beispielsweise peroral wie in Kapseln oder Tabletten, parenteral in Form steriler Lösungen oder Suspensionen sowie in einigen Fällen intravenös in Form steriler Lösungen. Die Endprodukte selbst sind zwar als freie Basen wirksam, doch kann man sie aus Gründen der Stabilität, Bequemlichkeit oder Kristallisation und erhöhter Löslichkeit, und dergleichen formulieren und in Form ihrer physiologisch verträglichen Säureadditionssalze verabreichen.

Die erfindungsgemäßen Wirkstoffe lassen sich peroral verabreichen, beispielsweise mit einem inerten Streckmittel oder eßbaren Träger, oder sie können in Gelatinekapseln eingeschlossen oder zu Tabletten gepreßt werden.

Zur peroralen therapeutischen Verabreichung kann man die erfindungsgemäßen Wirkstoffe in Vehikel einarbeiten und in Form von Tabletten, Pastillen, Kapseln, Elixieren, Suspensionen, Sirupen, Oblaten, Kaugummi und dergleichen anwenden. Diese Präparate sollen mindestens 0,5% Wirkstoff enthalten, aber dies läßt sich in Abhängigkeit von der jeweiligen Form variieren und kann zweckmäßig zwischen 4% und etwa 70% des Einheitsgewichts liegen. Bevorzugte Zusammensetzungen und Präparate gemäß vorliegender Erfindung werden so zubereitet, daß eine perorale Dosiseinheit 1,0—300 Milligramm Wirkstoff enthält.

Die Tabletten, Pillen, Kapseln, Pastillen und dergleichen können ferner folgende Bestandteile enthalten: ein Bindemittel wie mikrokristalline Cellulose, Traganthgummi oder Gelatine; ferner ein Trägerstoff wie Stärke oder Milchzucker, ein Sprengmittel wie Alginsäure, Maisstärke und dergleichen; ein Gleitmittel wie Magnesiumstearat oder kolloidales Siliciumdioxyd; ferner kann man einen Süßstoff wie Rohrzucker oder Saccharin oder auch einen Geschmacksstoff wie Pfefferminz, Methylsalicylat oder Orangenaroma zusetzen. Ist die Dosiereinheitsform eine Kapsel, so kann diese neben Stoffen der obigen Art einen flüssigen Träger wie ein Öl enthalten. Andere Dosiereinheitsformen können weitere verschiedene Stoffe enthalten, welche die physikalische Form der Dosiereinheit modifizieren, wie beispielsweise Überzüge. Tabletten oder Pillen können somit mit Zucker oder Schellack überzogen oder mit anderen erst im Darm löslichen Überzügen versehen werden. Ein Sirup kann neben den Wirkstoffen Rohrzucker als Süßstoff sowie gewisse Konservierungsmittel, Farbstoffe und Farben sowie Geschmacksstoffe enthalten. Bei der Bereitung dieser verschiedenen Zusammensetzungen verwendete Stoffe sollen pharmazeutisch rein und in den verwendeten Mengen nichttoxisch sein.

Zur parenteralen therapeutischen Verabreichung können die erfindungsgemäßen Wirkstoffe in eine Lösung oder Suspension eingearbeitet werden. Diese Zubereitungen sollen mindestens 0,1% Wirkstoff enthalten, doch läßt sich dies zwischen 0,5 und etwa 30% variieren. Bevorzugte Zusammensetzungen und Präparate gemäß vorliegender Erfindung werden so hergestellt, daß eine

parenterale Dosiseinheit 0,5 bis 100 Milligramm Wirkstoff enthält.

Die Lösungen oder Suspensionen können ferner folgende Bestandteile umfassen: ein steriles Verdünnungsmittel wie Wasser pro injectionem, physiologische Kochsalzlösung, nicht-flüchtige Öle, Polyäthylenglykole, Glyzerin, Propylenglykol oder sonstige synthetischen Lösungsmittel; antibakterielle Mittel wie Benzylalkohol; Antioxydantien wie Ascorbinsäure oder Natriumbisulfit; Chelatbildner wie Äthylendiamintetraessigsäure; Puffer wie Acetate, Zitrate oder Phosphate sowie Mittel zur Einstellung des osmotischen Drucks wie Kochsalz oder Dextrose. Das parenterale Präparat kann in Ampullen, zum einmaligen Gebrauch bestimmten Spritzen oder Mehrfachdosis-phiolen aus Glas oder Kunststoff eingeschmolzen werden.

## Beispiel 1

Man läßt ein Gemisch aus 1,6 g 10,11-Dihydro-10-hydroxydibenz[b,f]oxepin, 2,2 g $\beta$-Dimethylamino-äthylthiol-hydrochlorid und 4 ml Bortrifluoridätherat in 8 ml Eisessig 64 Stunden bei Raumtemperatur stehen und gibt dieses tropfenweise unter Rühren in kalte 20%ige Natronlauge. Das freigesetzte Amin wird mit Äther extrahiert, nacheinander mit Natronlauge und gesättigter Kochsalzlösung gewaschen und getrocknet. Der Äther wird bei vermindertem Druck entfernt, wobei ein dickflüssiges Öl verbleibt, welches man in Aceton auflöst und in sein kristallines Oxalat umwandelt. Das Salz wird aus Methanol/Aceton umkristallisiert, wobei farblose Kristalle, Schmelzpunkt 168—169° C, von 10,11-Dihydro-10-[$\beta$-(dimethylamino)-äthylthio]-dibenz[b,f]oxepin-oxalat zurückbleiben.

Analyse:

| | | | | |
|---|---|---|---|---|
| Berechnet für $C_{18}H_{21}NOS \cdot C_2H_2O_4$: | C 61,68%; | H 5,95%; | N 3,59%; | S 8,23%. |
| Gefunden: | C 61,50%; | H 6,01%; | N 3,54%; | S 8,38%. |

## Beispiel 2

Man läßt die Lösung von 2,5 g 10,11-Dihydro-10-hydroxydibenz[b,f]oxepin, 4,01 g $\beta$-Diäthylamino-äthylthiol-hydrochlorid und 8 ml Bortrifluoridätherat in 13 ml Eisessig 48 Stunden stehen, engt ein und gießt dann in kalte 25%ige Natronlauge. Das so erhaltene Öl wird mit Äther extrahiert. Man vereinigt die Ätherextrakte, wäscht nacheinander mit verdünnter Natronlauge und Wasser und trocknet danach. Die getrocknete Lösung wird filtriert und das Filtrat zur Trockne eingedampft, wobei ein Öl zurückbleibt. Man rührt das Öl mit 40%iger Natronlauge, extrahiert dann mit Äther und trocknet. Die ätherische Lösung wird filtriert und der Äther entfernt, wobei wiederum ein Öl verbleibt, das man durch eine Silikagelsäure mit 20% Methanol in Chloroform als Eluiermittel chromatographiert. Das chromatographierte Produkt wird in sein Oxalat, 10-[$\beta$-(Diäthylamino)-äthylthio]-10,11-dihydrodibenz[b,f]oxepinoxalat, ein weißes Salz vom Schmelzpunkt 109—111° C, überführt.

Analyse:

| | | | |
|---|---|---|---|
| Berechnet für $C_{20}H_{24}NOS \cdot C_2H_2O_4$: | C 63,44%; | H 6,29%; | N 3,36%. |
| Gefunden: | C 63,54%; | H 6,57%; | N 3,23%. |

## Beispiel 3

Man gibt die Lösung von 0,55 g 10,11-Dihydro-10-[$\beta$-(dimethylamino)-äthylthio]-dibenz[b,f]oxepin, die freie Base aus Beispiel 1, in 10 ml Chloroform tropfenweise zur Lösung von 0,28 g Bromcyan und 0,6 g Kaliumcarbonat in 5 ml Chloroform. Nach beendeter Zugabe läßt man 10 Minuten stehen und filtriert dann. Das Filtrat wird zur Trockne eingeengt, wobei ein dickflüssiges Öl verbleibt, welches beim Stehen kristallisiert. Die Kristallmasse wird aus niedrigsiedendem Petroläther umkristallisiert, was farblose Nadeln, Schmelzpunkt 77—78° C, von 10-($\beta$-Bromäthylthio)-10,11-dihydrodibenz[b,f]oxepin liefert.

Analyse:

| | | | |
|---|---|---|---|
| Berechnet für $C_{16}H_{15}BrOS$: | C 57,32%; | H 4,51%; | Br 23,84%. |
| Gefunden: | C 57,58%; | H 4,57%; | Br 24,20%. |

Beispiel 4

Man rührt ein Gemisch aus 1,5 g 10-($\beta$-Bromäthylthio)-10,11-dihydrodibenz[b,f]oxepin, Beispiel 3, 0,5 g N-Methylpiperazin, 1,0 g Natriumbicarbonat und 1,0 g Kaliumjodid in 15 ml Dimethylformamid 16 Stunden bei 80°C. Vor Verdünnen mit Wasser läßt man abkühlen. Das zweiphasige Gemisch extrahiert man dreimal mit je 100 ml Äther, vereinigt die Ätherextrakte und schüttelt gründlich mit einem großen Überschuß 2 n-Salzsäure. Die saure Lösung wird zur Freisetzung des Amins alkalisch gemacht. Man extrahiert das Amin mit Benzol, trocknet die Benzollösung und entfernt das Benzol im Vakuum, wobei ein blaßgelbliches Öl verbleibt. Das Öl wird in Äther gelöst und in ein kristallines Dihydrobromid überführt, welches man aus Methanol/Aceton umkristallisiert, was weiße Nadeln, Schmelzpunkt 180—182,5°C, von 10,11-Dihydro-10-[$\beta$-(4-methylpiperazinyl-1)-äthylthio]-dibenz[b,f]-oxepin-dihydrobromid liefert.

Analyse:

| | | | |
|---|---|---|---|
| Berechnet für $C_{21}H_{21}N_2OS \cdot 2\,HBr$: | C 48,84%; | H 5,46%; | N 5,42%. |
| Gefunden: | C 48,93%; | H 5,66%; | N 5,29%. |

Beispiel 5

Man rührt ein Gemisch aus 1,5 g 10-($\beta$-Bromäthylthio)-10,11-dihydrodibenz[b,f]oxepin, Beispiel 3, 0,5 g Morpholin, 1,0 g Natriumbicarbonat und 1,0 g Kaliumjodid in 15 ml Dimethylformamid 64 Stunden bei 60—70°C. Vor Verdünnen mit Wasser läßt man abkühlen. Das zweiphasige Gemisch extrahiert man mit Äther und schüttelt die vereinigten Ätherextrakte mit einem großen Überschuß 2 n-Salzsäure. Die saure Lösung wird mit Kaliumcarbonat alkalisch gemacht, wobei das Amin als Öl freigesetzt wird, welches man mit Äther extrahiert. Die ätherische Lösung wird getrocknet und der Äther im Vakuum entfernt, wobei wieder ein Öl entsteht, das in sein Oxalat umgewandelt wird. Das Salz wird als Methanol/Aceton/Äther umkristallisiert, was farblose Plättchen, Schmelzpunkt 196—198°C, von 10,11-Dihydro-10-($\beta$-morpholinoäthylthio)-dibenz[b,f]oxepin-oxalat liefert.

Analyse:

| | | | |
|---|---|---|---|
| Berechnet für $C_{20}H_{23}NO_2S \cdot C_2H_2O_4$: | C 61,23%; | H 5,84%; | N 3,26%. |
| Gefunden: | C 60,77%; | H 5,88%; | N 3,20%. |

Beispiel 6

Man behandelt eine Probe von 3,8 g 2-Chlor-10,11-dihydro-11-hydroxydibenz[b,f]oxepin ähnlich wie bei der Arbeitsweise nach Beispiel 1 mit 3,3 g $\beta$-Dimethylaminoäthylthiohydrochlorid, was grobkristallines 2-Chlor-10,11-dihydro-11-[$\beta$-(dimethylamino)-äthylthio]-dibenz[b,f]oxepin-oxalat vom Schmelzpunkt 139—141°C liefert.

Analyse:

| | | | |
|---|---|---|---|
| Berechnet für $C_{18}H_{28}ClNOS \cdot C_2H_2O_4$: | C 56,66%; | H 5,23%; | N 3,31%. |
| Gefunden: | C 56,58%; | H 5,27%; | N 3,31%. |

Beispiel 7

Man läßt die Lösung von 2,0 g 10,11-Dihydro-10-oxodibenz[b,f]oxepin, 2,96 g $\gamma$-Dimethylaminopropylthiol-hydrochlorid und 8 ml Bortrifluoridätherat in 8 ml Eisessig 16 Stunden stehen, erhitzt 30 Minuten auf dem Dampfbad zum Rückfluß, kühlt ab und gießt in 6 n-Natronlauge. Man extrahiert mit Äther, wäscht die vereinigten Ätherextrakte nacheinander mit 25%iger Natronlauge und Wasser und trocknet. Die getrocknete Lösung wird filtriert und das Filtrat eingeengt, wobei ein Öl verbleibt. Das Öl chromatographiert man durch eine Silikagelsäure mit 10% Methanol in Chloroform als Eluiermittel. Das chromatographierte Öl wird in sein Oxalsäuresalz, 10-[$\gamma$-(Dimethylamino)-propylthio]-dibenz[b,f]oxepin-oxalat vom Schmelzpunkt 151—152°C, umgewandelt.

Analyse:

| | | | |
|---|---|---|---|
| Berechnet für $C_{19}H_{21}NOS \cdot C_2H_2O_4$: | C 62,82%; | H 5,77%; | N 3,49%. |
| Gefunden: | C 62,71%; | H 5,77%; | N 3,43%. |

Beispiel 8

Man läßt ein Gemisch aus 1,2 g 10,11-Dihydro-10-oxodibenz[b,f]oxepin, 2,4 g $\beta$-Dimethylaminoäthylthiol-hydrochlorid und 2 ml Bortrifluoridätherat in 10 ml Eisessig 16 Stunden bei Raumtemperatur stehen und erwärmt 30 Minuten auf dem Dampfbad. Man gießt noch warm auf 200 g Eiswasser und macht das verdünnte Gemisch mit 40%iger Natronlauge alkalisch, wobei ein Öl freigesetzt wird, das man im Äther auflöst. Die Ätherlösung wird getrocknet und eingeengt, wobei ein dickflüssiges Öl verbleibt, das man in Äther in ein kristallines Oxalsäuresalz überführt. Das Salz wird aus Methanol/Äther umkristallisiert, was grobkristallines 10-[$\beta$-(Dimethylamino)-äthylthio]-dibenz[b,f]-oxepin-oxalat vom Schmelzpunkt 147—148° C liefert.

Analyse:

| | | | | |
|---|---|---|---|---|
| Berechnet für $C_{18}H_{19}NOS \cdot C_2H_2O_4$: | C 62,00%; | H 5,46%; | N 3,61%; | S 8,28%. |
| gefunden: | C 61,88%; | H 5,46%; | N 3,55%; | S 8,22%. |

Beispiel 9

Man versetzt die Lösung von 0,36 g Bromcyan und 2,28 g Kaliumcarbonat in 10 ml Chloroform unter Rühren tropfenweise mit der Lösung von 1,0 g 10-[$\beta$-(Dimethylamino)-äthylthio]-dibenz[b,f]oxepin, Beispiel 8, in Chloroform. Nach beendeter Zugabe erhitzt man 2 Stunden zum Rückfluß und entfernt das Lösungsmittel, wobei ein Öl verbleibt. Man löst das Öl in 10 ml Methanol, erhitzt die methanolische Lösung 10 Minuten zum Rückfluß und engt nochmals zur Gewinnung des Öls ein. Das Öl wird durch eine Silikagelsäule mit Äther als Eluiermittel chromatographiert. Man fängt die Kopffraktion auf und engt ein, wobei 10-[$\beta$-(Brom)-äthylthio]-dibenz[b,f]oxepin als weißer Feststoff vom Schmelzpunkt 106—107° C verbleibt.

Analyse:

| | | | | |
|---|---|---|---|---|
| Berechnet für $C_{16}H_{13}BrOS$: | C 57,66%; | H 3,93%; | S 9,62%; | Br 23,98%. |
| Gefunden: | C 58,38%; | H 3,89%; | S 9,84%; | Br 24,12%. |

Beispiel 10

Man behandelt die Lösung von 2,0 g 10,11-Dihydro-10-oxodibenz[b,f]oxepin, 3,76 g $\beta$-Diisopropylaminoäthylthiolhydrochlorid und 8 ml Bortrifluoridätherat in 10 ml Eisessig gemäß Beispiel 7, was ein gelbes Öl liefert. Man chromatographiert das Öl durch eine Silikagelsäure mit 5% Methanol in Chloroform als Eluiermittel. Beim Kratzen erstarrt das chromatographierte Öl zu einem blaßgelben Pulver, Schmelzpunkt 65—66° C, von 10-[$\beta$-(Diisopropylamino)-äthylthio]-dibenz[b,f]oxepin.

Analyse:

| | | | |
|---|---|---|---|
| Berechnet für $C_{22}H_{27}NOS$: | C 74,74%; | H 7,70%; | N 3,96%. |
| Gefunden: | C 74,95%; | H 7,71%; | N 4,06%. |

Beispiel 11

Man rührt ein Gemisch aus 1,14 g 10-[$\beta$-(Brom)-äthylthio]-dibenz[b,f]oxepin, Beispiel 9, 0,36 g Morpholin, 0,92 g Natriumbicarbonat und 0,93 g Kaliumjodid in 15 ml Dimethylformamid 16 Stunden, wobei man das Gemisch auf 60 bis 65° C hält. Danach gießt man in 150 ml Wasser, extrahiert das zweiphasige Gemisch mit Äther, vereinigt die Ätherextrakte und trocknet. Die getrocknete Ätherlösung wird filtriert und das Filtrat eingeengt, wobei das Öl verbleibt, das man durch eine Silikagelsäule mit 5% Methanol in Chloroform als Eluiermittel chromatographiert. Man überführt das

chromatographierte Öl in sein weißes Oxalsäuresalz, 10-[β-(Morpholino)-äthylthio]-dibenz[b,f]oxepin-oxalat vom Schmelzpunkt 181—183° C.

Analyse:

| | | | |
|---|---|---|---|
| Berechnet für $C_{20}H_{21}NO_2S \cdot C_2H_2O_4$: | C 61,52%; | H 5,40%; | N 3,26%. |
| Gefunden: | C 61,33%; | H 5,35%; | N 3,34%. |

Beispiel 12

Man versetzt die Lösung von 2,08 g 10-[β-(Dimethylamino)-äthylthio]-dibenz[b,f]oxepin, der freien Base aus Beispiel 8, in 10 ml Methylenchlorid tropfenweise mit der Lösung von 1,26 g Chlorameisensäurephenylester in 10 ml Methylenchlorid. Nach beendeter Zugabe läßt man 16 Stunden bei Raumtemperatur weiterrühren und engt dann ein, wobei ein öliger Rückstand verbleibt. Man verreibt den Rückstand mit Hexan und kühlt dann zwecks Erstarrung auf −20° C ab. Der Feststoff wird aus Äther/Hexan umkristallisiert, was weiße Nadeln, Schmelzpunkt 103—103,5° C, von 10-[β-(N-Methyl-N-phenoxycarbonyl)-aminoäthylthio]-dibenz[b,f]oxepin liefert.

Analyse:

| | | | |
|---|---|---|---|
| Berechnet für $C_{24}H_{21}NO_3S$: | C 71,42%; | H 5,24%; | N 3,47%. |
| Gefunden: | C 71,37%; | H 5,31%; | N 3,51%. |

Beispiel 13

Man erhitzt eine Suspension von 1,6 g 10-[β-(N-Methyl-N-phenoxycarbonylamino)-äthylthio]-dibenz[b,f]oxepin, Beispiel 12, und 3,4 g Kaliumhydroxydplätzchen in 40 ml Äthylenglykol im Verlauf von 60 Minuten auf 150 bis 160° C. Man rührt noch 30 Minuten bei dieser Temperatur weiter. Man kühlt ab, verdünnt mit Wasser und extrahiert das zweiphasige Gemisch mit überschüssigem Äther. Die Ätherextrakte werden vereinigt, mit Wasser gewaschen und getrocknet und das Lösungsmittel entfernt, wobei ein klares dünnflüssiges Öl verbleibt. Man überführt das Öl in sein Oxalsäuresalz und kristallisiert aus Methanol um, was glänzende Nadeln, Schmelzpunkt 207—208° C (Zersetzung), von 10-[β-(Methylamino)-äthylthio]-dibenz[b,f]oxepin-oxalat liefert.

Analyse:

| | | | |
|---|---|---|---|
| Berechnet für $C_{17}H_{17}NOS \cdot C_2H_2O_4$: | C 61,11%; | H 5,12%; | N 3,75%. |
| Gefunden: | C 60,85%; | H 5,14%; | N 3,78%. |

Beispiel 14

Man rührt ein Gemisch aus 1,82 g 10-[β-(Methylamino)-äthylthio]-dibenz[b,f]oxepin, der freien Base aus Beispiel 13, 0,72 g Cyclopropylmethylchlorid, 1,93 g Natriumbicarbonat und einigen Kristallen Kaliumjodid in 50 ml Dimethylformamid 16 Stunden bei 80° C. Man läßt das Reaktionsgemisch abkühlen, filtriert und engt das Filtrat ein, wobei ein Öl verbleibt, das man in das Oxalsäuresalz, Schmelzpunkt 137—139° C, von 10-[β-(N-Cyclopropylmethyl-N-methylamino)-äthylthio]-dibenz[b,f]-oxepin überführt.

Analyse:

| | | | |
|---|---|---|---|
| Berechnet für $C_{21}H_{23}NOS \cdot C_2H_2O_4$: | C 64,61%; | H 5,89%; | N 3,28%. |
| Gefunden: | C 64,80%; | H 5,96%; | N 3,24%. |

Beispiel 15

Man rührt ein Gemisch aus 1,5 g 10-[β-(Methylamino)-äthylthio]-dibenz[b,f]oxepin, der freien Base aus Beispiel 13, 1,1 g Äthyljodid, 1,48 g Natriumbicarbonat und 1,46 g Kaliumjodid in 20 ml

Dimethylformamid 16 Stunden bei 80—85° C. Man läßt das Reaktionsgemisch abkühlen, verdünnt mit 75 ml Wasser und extrahiert dann dreimal mit je 75 ml Äther. Die Ätherextrakte werden vereinigt, mit 75 ml gesättigter Kochsalzlösung gewaschen und dann getrocknet. Man filtriert die getrocknete Lösung und engt das Filtrat ein, wobei ein Öl verbleibt. Man chromatographiert das Öl durch eine Aluminiumoxydsäule mit Äther als Aluiermittel. Das chromatographierte Öl wird in sein Oxalsäuresalz umgewandelt und dieses aus Aceton/Äther umkristallisiert, was 10-[$\beta$-(N-Äthyl-N-methylamino)-äthylthio]-dibenz[b,f]oxepinoxalat als weißes Produkt vom Schmelzpunkt 126—128° C liefert.

Analyse:

| | | | | |
|---|---|---|---|---|
| Berechnet für $C_{19}H_{21}NOS \cdot C_2H_2O_4$: | C 62,82%; | H 5,77%; | N 3,49%. |
| Gefunden: | C 62,39%; | H 5,69%; | N 3,56%. |

Beispiel 16

Verfährt man wie in Beispiel 15 unter Ersatz des Äthyljodids durch Propargylbromid, so erhält man 10-[$\beta$-(N-Methyl-N-propargyl)-aminoäthylthio]-dibenz[b,f]oxepin-oxalat als weißen Feststoff vom Schmelzpunkt 140—142° C.

Analyse:

| | | | |
|---|---|---|---|
| Berechnet für $C_{20}H_{19}NOS \cdot C_2H_2O_4$: | C 64,21%; | H 5,14%; | N 3,40%. |
| Gefunden: | C 64,49%; | H 5,22%; | N 3,41%. |

Beispiel 17

Man versetzt ein Gemisch aus 2,8 g $\beta$-Diäthylaminoäthylthiolhydrochlorid in 5 ml Essigsäure und 5 ml Bortrifluoridätherat bei einer Temperatur von 0° C tropfenweise mit der Lösung von 2,0 g 2-Chlor-10,11-dihydroxydibenz[b,f]oxepin in 6 ml Eisessig. Nach beendeter Zugabe rührt man 20 Minuten bei 0° C und danach 16 Stunden bei Raumtemperatur. Das gut durchgerührte Gemisch wird bei 0° C langsam zu 50 ml 20%iger Natronlauge gegeben. Nach dieser Zugabe setzt man Äther dazu, filtriert das zweiphasige Gemisch und läßt es sich in Schichten scheiden. Die wäßrige Schicht wird zweimal mit je 50 ml Äther extrahiert und die Ätherextrakte mit der organischen (ätherischen) Phase vereinigt. Die vereinigten Lösungen wäscht man nacheinander je einmal mit 40 ml wäßriger Natronlauge, 30 ml 10%iger Natronlauge, 50 ml Wasser und 30 ml gesättigter Kochsalzlösung und trocknet dann über Kaliumcarbonat und Kaliumhydroxydplättchen, wobei ein Öl verbleibt. Das Öl wird mit 5% Methanol/Chlororoform durch eine Silikagelsäure chromatographiert, wobei man ein gereinigtes Öl erhält, das in Äther gelöst und mit ätherischer Oxalsäurelösung behandelt wird, um das entsprechende Oxalsäuresalz zu erhalten, welches beim Umkristallisieren aus Aceton 2-Chlor-11-[$\beta$-(diäthylamino)-äthylthio]-10,11-dihydrodibenz[b,f]oxepin-oxalat als weißes Pulver vom Schmelzpunkt 126,5—128,5° C ergibt.

Analyse:

| | | | | |
|---|---|---|---|---|
| Berechnet für $C_{20}C_{23}ClNOS \cdot C_2H_2O_4$: | C 58,60%; | H 5,59%; | N 3,11%; | Cl 7,86%. |
| Gefunden: | C 58,68%; | H 5,78%; | N 2,95%; | Cl 8,27%. |

Beispiel 18

Man versetzt die Lösung von 2,5 g 10,11-Dihydro-10-hydroxydibenz[b,f]oxepin und 3,7 g $\gamma$-Dimethylaminopropylthiolhydrochlorid in 13 ml Eisessig mit 8 ml Bortrifluoridätherat. Man läßt 24 Stunden stehen und gießt dann in 50 ml gekühlte 25%ige Natronlauge. Man extrahiert das basische Gemisch mit Äther, vereinigt die Ätherextrakte, wäscht diese nacheinander mit 20%iger Natronlauge und Wasser, trocknet, filtriert und engt das Filtrat ein, wobei ein Öl verbleibt. Das Öl wird durch eine Silikagelsäule mit 5% Metahnol in Chloroform als Eluiermittel chromatographiert und das Eluat eingedampft, wobei ein gereinigtes Öl verbleibt, das in ein weißes grobkristallines Oxalsäuresalz, Schmelzpunkt 179—181° C, von 10,11-Dihydro-10-[$\gamma$-(dimethylamino)-propylthio]dibenz[b,f]oxepin überführt wird.

Analyse:

| | | | |
|---|---|---|---|
| Berechnet für $C_{19}H_{22}NOS \cdot C_2H_2O_4$: | C 62,66%; | H 6,01%; | N 3,48%. |
| Gefunden: | C 62,60%; | H 6,26%; | N 3,64%. |

Beispiel 19

Man perlt Äthylamin 5 Minuten lang in ein Gemisch aus 1,5 g 10-[(β-Bromäthyl)-thio]-10,11-dihydrodibenz[b,f]oxepin, Beispiel 3, und 1,0 g Kaliumjodid in 15 ml Dimethylformamid. Man läßt auf Raumtemperatur abkühlen und rührt dann 16 Stunden. Nach Zusatz von Eiswasser extrahiert man das zweiphasige Gemisch dreimal mit Benzol, trocknet die vereinigten Benzolextrakte und dampft zur Trockne ein, wobei ein Rohprodukt verbleibt, welches man in Aceton in sein Oxalsäuresalz überführt. Das Salz wird aus 90%igem Äthanol umkristallisiert, was 10-[β-(Äthylamino)-äthylthio]-10,11-dihydrodibenz[b,f]oxepin-oxalat als farblose Prismen vom Schmelzpunkt 205—207° C, Zersetzung, ergibt.

Analyse:

| | | | |
|---|---|---|---|
| Berechnet für $C_{18}H_{21}NOS \cdot C_2H_2O_4$: | C 61,67%; | H 5,95%; | N 3,60%. |
| Gefunden: | C 61,68%; | H 5,97%; | N 3,39%. |

Beispiel 20

Man rührt die Lösung von 2,5 g 10,11-Dihydro-2-methylthio-11-oxodibenz[b,f]oxepin, 2,8 g β-Dimethylaminoäthylthiolhydrochlorid und 24 ml Eisessig 30 Minuten bei Raumtemperatur und gibt unter weiterem Rühren 8 ml Bortrifluoridätherat dazu. Nach beendeter Zugabe wird der Rührer abgestellt und das Reaktionsgemisch 72 Stunden stehengelassen, bevor man es in 50 ml Eiswasser gibt. Das verdünnte Gemisch wird mit 10%iger Natronlauge stark alkalisch gemacht und mit Äther extrahiert. Die vereinigten Ätherextrakte werden getrocknet und zur Trockne eingedampft, wobei ein Öl verbleibt. Man chromatographiert das Öl durch eine Silikagelsäule mit 5% Methanol in Chloroform als Eluiermittel, sammelt die gewünschten Fraktionen und engt ein, wobei ein gereinigtes Öl verbleibt. Dieses wird in das weiße grobkristalline Oxalsäuresalz von 11-[β-(Dimethylamino)-äthylthio]-2-methylthiodibenz[b,f]oxepin umgewandelt.

Analyse:

| | | |
|---|---|---|
| Berechnet für $C_{19}H_{21}NOS_2 \cdot C_2H_2O_4$: | C 58,18%; | H 5,35%. |
| Gefunden: | C 57,99%; | H 5,26%. |

Beispiel 21

Man versetzt die Lösung von 1,0 g 2-Fluor-10,11-dihydro-11-oxodibenz[b,f]oxepin und 1,2 g Dimethylaminoäthanthiolhydrochlorid in 11 ml Eisessig mit 3,3 ml Bortrifluoridätherat. Nach beendeter Zugabe rührt man 64 Stunden bei Raumtemperatur, gießt in 25 ml auf 0° C gekühlte 20%ige Natronlauge und extrahiert das wäßrige Gemisch mit Äther. Die vereinigten Ätherextrakte werden nacheinander zweimal mit 20%iger Natronlauge, einmal mit Wasser und einmal mit gesättigter Kochsalzlösung gewaschen und dann zu einem Öl getrocknet. Dieses chromatographiert man durch eine Silikagelsäule mit einer 5%igen Methanollösung in Chloroform, was ein gereinigtes Öl ergibt, welches man mit ätherischer Bromwasserstofflösung unter Bildung eines weißen Niederschlags behandelt. Der Niederschlag wird mit Äther gewaschen und aus Aceton umkristallisiert, was 2-Fluor-11-[β-(dimethylamino)-äthylthio]-dibenz[b,f]oxepin-hydrobromid als weißes Pulver vom Schmelzpunkt 197—198,5° C liefert.

Analyse:

| | | | | |
|---|---|---|---|---|
| Berechnet für $C_{18}H_{17}FNOS \cdot HBr$: | C 54,55%; | H 4,83%; | N 3,54%; | Br 20,17%. |
| Gefunden: | C 54,49%; | H 4,80%; | N 3,5%; | Br 20,14%. |

2-Fluor-10,11-dihydro-11-oxodibenz[b,f]oxepin, das Ausgangsmaterial für Beispiel 21, kann nach der folgenden Reaktionsfolge hergestellt werden:

a) Man gibt 57 ml Nitrobenzol zu 147 g Jodbenzoesäure und 45,5 g Kaliumcarbonat. Man erhitzt 40 Minuten unter Rühren bei 160° C. Das erhitzte Gemisch wird mit weiteren 46,5 g Kaliumcarbonat und dann nacheinander mit 73,1 g 4-Fluorphenol, nochmals 46,5 g Kaliumcarbonat und 0,3 g Kupferpulver versetzt. Nach diesen Zusätzen rührt man 45 Minuten bei 160° C und kühlt die so erhaltene Masse auf 0° C. Die abgekühlte Masse wird mit 100 ml Wasser und 220 ml 6 n-Salzsäure vermischt. Das saure Gemisch wird mit Wasser auf ein Volumen von einem Liter verdünnt und dann mit 450 ml Chloroform vermischt. Der weiße Feststoff wird abfiltriert und mit Chloroform und Wasser gewaschen. Man löst den Feststoff in heißem Aceton, kühlt ab und filtriert, wobei 2-(4-Fluorphenoxy)-benzoesäure als weißes kristallines Produkt vom Schmelzpunkt 146—147° C verbleibt.

b) Man versetzt 3,28 g 2-(4-Fluorphenoxy)-benzoesäure mit 5,6 ml 97%igem Thionylchlorid. Man erhitzt 10 Minuten auf dem Dampfbad und entfernt dann alles überschüssige Thionylchlorid bei vermindertem Druck. Der flüssige Rückstand wird in 30 ml 1,2-Dichloräthan gelöst und im Verlauf von 30 Minuten tropfenweise zu einem Gemisch von 1,9 g Aluminiumchlorid und 5 ml 1,2-Dichloräthan gegeben. Nach beendeter Zugabe rührt man 2 Stunden unter Rückfluß und läßt 64 Stunden bei Raumtemperatur stehen. Dann gießt man auf ein Gemisch von 150 ml Eis und Wasser sowie 125 ml Äther. Das zweiphasige Gemisch wird durch Papier filtriert und geschieden. Man sammelt die wäßrige Schicht und extrahiert zweimal mit Äther (je 50 ml). Die vereinigten Ätherextrakte werden nacheinander mit zweimal je 25 ml gesättigter Natriumbicarbonatlösung und einmal mit 25 ml gesättigter Kochsalzlösung gewaschen und dann zu einem Öl getrocknet. Das Öl wird mit Hexan behandelt und die so erhaltene Lösung abgegossen und eingedampft, wobei ein leuchtend gelber kristalliner Feststoff verbleibt. Dieser wird durch eine Silikagelsäule mit Chloroform als Eluiermittel chromatographiert und aus Cyclohexan umkristallisiert, was 2-Fluor-10,11-dihydro-11-oxodibenz[b,f]oxepin als gelblichweisen kristallinen Feststoff vom Schmelzpunkt 85,5—87,5° C liefert.

Die Ketonausgangsstoffe für andere Beispiele können in ähnlicher Weise wie oben erläutert hergestellt werden.

## Beispiel 22

Man rührt die Lösung von 2,5 g 2-Chlor-10,11-dihydro-11-oxodibenz[b,f]oxepin und 2,9 g β-Dimethylaminoäthylthiolhydrochlorid in 25 ml Eisessig 30 Minuten bei Raumtemperatur und versetzt unter fortlaufendem Rühren mit 5 ml Bortrifluoridätherat. Nach beendeter Zugabe wird der Rührer abgestellt und das Reaktionsgemisch 24 Stunden stehengelassen. Man macht mit 10%iger Natronlauge alkalisch und extrahiert mit Äther. Die vereinigten Ätherextrakte werden getrocknet und filtriert und das Lösungsmittel dann entfernt, wobei ein Öl verbleibt. Dieses chromatographiert man durch eine Silikagelsäule mit 5% Methanol in Chloroform als Eluiermittel, wobei man ein gereinigtes Öl erhält, das in ein weißes Oxalsäuresalz, Schmelzpunkt 183—184° C, von 2-Chlor-11-[β-(dimethylamino)-äthylthio]-dibenz-[b,f]oxepin überführt wird.

Analyse:

| | | |
|---|---|---|
| Berechnet für $C_{18}H_{18}ClNOS \cdot C_2H_2O_4$: | C 56,93%; | H 4,78%. |
| Gefunden: | C 56,67%; | H 4,70%. |

## Beispiel 23

Man rührt die Lösung von 2,5 g 10,11-Dihydro-2-(methylthio)-11-oxodibenz[b,f]oxepin und 3,33 g β-Diäthylaminoäthylthiolhydrochlorid in 24 ml Eisessig 30 Minuten und versetzt mit 8 ml Bortrifluoridätherat. Nach dieser Zugabe läßt man 72 Stunden stehen, bevor man in 50 ml Eiswasser gießt. Das verdünnte Gemisch wird mit 10%iger Natronlauge stark alkalisch gemacht und dann mit Äther extrahiert. Die vereinigten Ätherextrakte werden getrocknet, filtriert und der Äther abgedampft, wobei ein gelbes Öl verbleibt. Man chromatographiert das Öl durch eine Säule von Silikagel in Chloroform mit 5% Methanol in Chloroform als Eluiermittel, um das Öl zu reinigen, welches in sein Bromwasserstoffsäuresalz umgewandelt und aus Aceton umkristallisiert wird, was das Salz 11-[β-(Dimethylamino)-äthylthio]-2-(methylthio)-dibenz[b,f]oxepin-hydrobromid vom Schmelzpunkt 184—186° C liefert.

Analyse:

| | | | |
|---|---|---|---|
| Berechnet für $C_{21}H_{25}NOS_2 \cdot HBr$: | C 55,74%; | H 5,79%; | N 3,10%. |
| Gefunden: | C 55,83%; | H 5,85%; | N 3,05%. |

Beispiel 24

Man versetzt die Lösung von 11,3 g 11-[β-(Dimethylamino)-äthylthio]-2-(methylthio)-dibenz[b,f]oxepin, der freien Base aus Beispiel 21, und 10,0 g Kaliumcarbonat in 50 ml Methylenchlorid unter Rühren tropfenweise mit der Lösung von 5,7 g Chlorameisensäurephenylester in 50 ml Methylenchlorid. Nach beendeter Zugabe rührt man 24 Stunden bei Raumtemperatur und dampft dann zur Trockne ein. Der Rückstand wird mit Äther angerieben und anschließend die ätherische Lösung nacheinander mit 10%iger Natronlauge und Wasser gewaschen, getrocknet und filtriert und das Filtrat zur Trockne eingedampft, wobei ein gelbes Öl verbleibt. Dieses chromatographiert man durch eine Silikagelsäule mit Methylenchlorid als Eluiermittel, wobei man 11-[β-(N-Methyl-N-phenoxycarbonylamino)-äthylthio]-2-(methylthio)-dibenz[b,f]-oxepin als gereinigtes orangefarbenes Öl erhält.

Analyse:

| | | | |
|---|---|---|---|
| Berechnet für $C_{25}H_{23}NO_3S_2$: | C 66,79%; | H 5,16%; | N 3,12%. |
| Gefunden: | C 66,84%; | H 4,93%; | N 3,06%. |

Beispiel 25

Man rührt ein Gemisch aus 8,3 g 11-[β-(N-Methyl-N-phenoxycarbonyl)-aminoäthylthio]-2-(methylthio)-dibenz[b,f]oxepin, Beispiel 24, 190 ml Äthylenglykol und 16 g Kaliumhydroxyd 30 Minuten bei 155°C. Man kühlt ab und gießt dann auf 500 ml Eiswasser. Das zweiphasige Gemisch wird mit 1 : 1 Äther/Benzol extrahiert, und die vereinigten Extrakte werden mit Wasser gewaschen und getrocknet. Man filtriert die getrocknete Lösung und dampft das Filtrat zur Trockne ein, wobei ein Öl verbleibt. Dieses wird in sein Maleinsäuresalz umgewandelt und aus Aceton umkristallisiert, wobei das Salz 11-[β-(Methylamino)-äthylthio]-2-(methylthio)-dibenz[b,f]oxepin-maleinat vom Schmelzpunkt 160—162°C verbleibt.

Analyse:

| | | | |
|---|---|---|---|
| Berechnet für $C_{18}H_{19}NOS_2 \cdot C_4H_4O_4$: | C 59,30%; | H 5,20%; | N 3,14%. |
| Gefunden: | C 59,40%; | H 5,23%; | N 3,08%. |

Beispiel 26

Man rührt die Reaktionslösung von 1,9 g 11-[β-(Methylamino)-äthylthio]-2-(methylthio)-dibenz[b,f]oxepin, der freien Base aus Beispiel 26, 1,2 g Äthyljodid und 1,60 g Natriumbicarbonat in 25 ml Dimethylformamid 72 Stunden unter Erwärmen. Man verdünnt mit 100 ml Wasser und extrahiert das zweiphasige Gemisch mit Äther. Die vereinigten Ätherextrakte werden mit Wasser gewaschen, getrocknet und dann filtriert und das Filtrat zur Trockne eingedampft, wobei ein Öl verbleibt. Das Öl wird in das weiße Salz 11-[β-(Äthylmethylamino)-äthylthio]-2-(methylthio)-dibenz[b,f]oxepinmaleinat vom Schmelzpunkt 112—114°C überführt.

Analyse:

| | | | |
|---|---|---|---|
| Berechnet für $C_{20}H_{23}NOS_2 \cdot C_4H_4O_4$: | C 60,86%; | H 5,75%; | N 2,96%. |
| Gefunden: | C 61,11%; | H 5,74%; | N 2,87%. |

Beispiele 27 und 28

Man behandelt 2-Fluor-11-[β-(dimethylamino)-äthylthio]-dibenz[b,f]oxepin, die freie Base aus Beispiel 22, bzw. 2-Chlor-10,11-dihydro-11-[β-(dimethylamino)-äthylthio]-dibenz[b,f]oxepin, die freie Base aus Beispiel 6, gemäß der Arbeitsweise von Beispiel 25, wobei man in Beispiel 27 2-Fluor-11-[β-(N-methyl-N-phenoxycarbonylamino)-äthylthio]-dibenz[b,f]oxepin bzw. in Beispiel 28 2-Chlor-11-[β-(N-methyl-N-phenoxycarbonylamino)-äthylthio]-dibenz[b,f]oxepin erhält.

Analyse: [Beispiel 27]

| | | | | |
|---|---|---|---|---|
| Berechnet für $C_{24}H_{20}FNO_3S$: | C 68,40%; | H 4,78%; | N 3,32%; | F 4,51%. |
| Gefunden: | C 68,25%; | H 4,85%; | N 3,28%; | F 4,80%. |

Beispiel 29

Man rührt die Reaktionslösung von 5,1 g 2-Fluor-11-[$\beta$-(N-methyl-N-phenoxycarbonylamino)-äthylthio]-dibenz[b,f]oxepin und 10,6 g Kaliumhydroxyd in 125 ml Äthylenglykol 30 Minuten unter Erhitzen von 80 auf 155° C und danach 30 Minuten bei 155° C. Danach läßt man 16 Stunden bei 0° C stehen, bevor man auf 350 ml Eiswasser gießt. Das zweiphasige Gemisch wird dreimal mit je 125 ml 1 : 1 Äther/Benzol extrahiert. Man wäscht die vereinigten organischen Schichten nacheinander dreimal mit Wasser und einmal mit gesättigter Kochsalzlösung und trocknet dann, wobei ein klares orangefarbenes Öl verbleibt. Das Öl wird mit einer Portion von 50 ml sowie einer von 0,25 ml siedendem Hexan umgeschwenkt und von irgendwelchem Rückstand, der das Öl gelb macht, abgegossen. Man behandelt das Öl in Äther mit ätherischer Maleinsäure, um ein Salz zu bilden, das aus Methanol/Aceton/Äther umkristallisiert wird, was 2-Fluor-11-[$\beta$-(methylamino)-äthylthio]-dibenz[b,f]oxepin-maleinat als weißes Pulver vom Schmelzpunkt 135,5—136° C liefert.

Analyse:

| | | | | |
|---|---|---|---|---|
| Berechnet für $C_{17}H_{16}FNOS \cdot C_4H_4O_4$: | C 60,43%; | H 4,83%; | N 3,36%; | F 4,55%. |
| Gefunden: | C 60,42%; | H 4,86%; | N 3,26%; | F 4,85%. |

Beispiel 30

Man rührt das Reaktionsgemisch von 4,0 g 2-Chlor-11-[$\beta$-(N-methyl-N-phenoxycarbonylamino)-äthylthio]-dibenz[b,f]oxepin, Beispiel 28 und 8,5 g Kaliumhydroxyd in 80 ml Äthylenglykol 30 Minuten bei 155° C. Man läßt abkühlen und versetzt mit Eis. Man extrahiert mit Äther, trocknet die vereinigten Ätherextrakte und dampft den Äther ab, wobei ein Öl verbleibt. Dieses behandelt man in Äther mit ätherischer Maleinsäure und kristallisiert das so erhaltene Salz aus Methanol/Äther um, was 2-Chlor-11-[$\beta$-(methylamino)-äthylthio]-dibenz[b,f]oxepin-maleinat als farblose Prismen vom Schmelzpunkt 153—154° C liefert.

Analyse:

| | | | | |
|---|---|---|---|---|
| Berechnet für $C_{17}H_{16}ClNOS \cdot C_4H_4O_4$: | C 58,12%; | H 4,64%; | N 3,22%; | Cl 8,17%. |
| Gefunden: | C 58,15%; | H 4,77%; | N 3,24%; | Cl 8,33%. |

Beispiel 31

Man versetzt das Gemisch von 1,1 g $\beta$-Dimethylaminoäthanthiolhydrochlorid und 2,8 ml Bortrifluoridätherat in 5 ml Eisessig tropfenweise mit der Lösung von 0,9 g 2-Fluor-10,11-dihydro-11-hydroxydibenz[b,f]oxepin in 4,4 ml Eisessig. Nach beendeter Zugabe läßt man 16 Stunden rühren, bevor man auf ein Gemisch von 30 ml 20%iger Natronlauge und Eis gießt. Man extrahiert mit Äther, wäscht die vereinigten Ätherextrakte nacheinander zweimal mit 20%iger Natronlauge, einmal mit Wasser und einmal mit gesättigter Kochsalzlösung. Die gewaschenen Extrakte werden getrocknet, wobei ein Öl entsteht, welches man durch eine Silikagelsäule mit 5% Methanol in Chloroform als Eluiermittel chromatographiert, was ein gereinigtes Öl ergibt. Dieses wird in sein Oxalsäuresalz überführt, das man aus Aceton umkristallisiert, was 2-Fluor-10,11-dihydro-11-[$\beta$-(dimethylamino)-äthylthio]-dibenz[b,f]oxepin-oxalat als weißes Pulver vom Schmelzpunkt 169—170,5° C liefert.

Analyse:

| | | | | |
|---|---|---|---|---|
| Berechnet für $C_{18}H_{20}FNOS \cdot C_2H_2O_4$: | C 58,96%; | H 5,44%; | N 3,44%; | F 4,66%. |
| Gefunden: | C 59,17%; | H 5,44%; | N 3,51%; | F 4,81%. |

Beispiel 32

Man versetzt ein Gemisch von 2,3 g Bromcyan und 5,0 g Kaliumcarbonat in 40 ml Chloroform portionsweise im Verlauf von 50 Minuten mit 4,8 g 2-Fluor-10,11-dihydro-11-[β-(dimethylamino)-äthylthio]-dibenz[b,f]oxepin, der freien Base aus Beispiel 32, in 85 ml Chloroform. Nach beendeter Zugabe rührt man 20 Minuten vor dem Abfiltrieren. Das Filtrat wird eingedampft, wobei ein Öl verbleibt, welches man dreimal mit siedendem Hexan behandelt. Die vereinigten Hexanportionen werden eingedampft, wobei ein Öl verbleibt, welches man durch eine Silikagelsäule mit Methylenchlorid als Eluiermittel chromatographiert und aus kaltem Hexan umkristallisiert, was 11-[β-(Brom)-äthylthio]-2-fluor-10,11-dihydrodibenz[b,f]oxepin als weißes Pulver vom Schmelzpunkt 46—48° C liefert.

Analyse:

| | | | | |
|---|---|---|---|---|
| Berechnet für $C_{16}H_{14}BrFOS$: | C 54,41%; | H 4,00%; | Br 22,63%; | F 5,38%. |
| Gefunden: | C 54,36%; | H 4,01%; | Br 22,91%; | F 5,69%. |

Beispiel 33

Man perlt Methylamin 5 Minuten lang in 10 ml Dimethylsulfoxyd. Diese Lösung versetzt man tropfenweise mit der Lösung von 2,2 g 11-[β-(Bromäthyl)-thio]-2-fluor-10,11-dihydrodibenz-[b,f]oxepin in 11 ml Dimethylsulfoxid. Soviel Methylamin wird in das Reaktionsgemisch eingeperlt, daß man vollständige Umsetzung erzielt. Danach läßt man das Reaktionsgemisch 64 Stunden stehen, gießt auf 125 ml Eiswasser und extrahiert das zweiphasige Gemisch dreimal mit Äther. Die vereinigten Ätherextrakte werden nacheinander mit zweimal je 40 ml Wasser und einmal 10 ml gesättigter Kochsalzlösung gewaschen und unter Bildung eines Öls getrocknet. Man löst das Öl in Äther und behandelt mit ätherischer Maleinsäure, wodurch ein Salz ausfällt, das man aus Aceton/Äther umkristallisiert, wobei man 2-Fluor-10,11-dihydro-11-[β-(methylamino)-äthylthio]-dibenz[b,f]oxepin-maleinat als weißen Feststoff vom Schmelzpunkt 118—119,5° C erhält.

Analyse:

| | | | | |
|---|---|---|---|---|
| Berechnet für $C_{17}H_{18}FNOS \cdot C_4H_4O_4$: | C 60,14%; | H 5,29%; | N 3,34%; | F 4,53%. |
| Gefunden: | C 60,11%; | H 5,19%; | N 3,19‰; | F 4,80%. |

Beispiel 34

Man versetzt die Lösung von 2,8 g β-Dimethylaminoäthylthiolhydrochlorid und 7 ml Bortrifluoridätherat in 10 ml Eisessig unter Rühren tropfenweise mit der Lösung von 2,5 g 10,11-Dihydro-11-hydroxy-2-methylthiodibenz[b,f]oxepin in 10 ml Eisessig. Nach beendeter Zugabe läßt man 24 Stunden stehen, bevor man das Reaktionsgemisch in 50 ml kalte 25%ige Natronlauge gibt. Man extrahiert mit Äther, wäscht die vereinigten Ätherextrakte nacheinander mit 20%iger Natronlauge und Wasser und trocknet. Die getrocknete Lösung wird filtriert und das Filtrat zur Trockne eingedampft, wobei ein Öl verbleibt, daß man in Chloroform löst. Die Chloroformlösung wird durch eine Silikagelsäule mit 5% Methanol in Chloroform als Eluiermittel chromatographiert, wobei man ein gereinigtes Öl erhält. Man überführt das gereinigte Produkt in sein Maleinsäuresalz, ein weißes Pulver, Schmelzpunkt 100—102° C, aus 10,11-Dihydro-11-[β-(dimethylamino)-äthylthio]-2-(methylthio)-dibenz[b,f]oxepin-maleinat.

Analyse:

| | | | |
|---|---|---|---|
| Berechnet für $C_{19}H_{23}NOS_2 \cdot C_4H_4O_4$: | C 59,84%; | H 5,90%; | N 3,04%. |
| Gefunden: | C 59,92%; | H 5,93%; | N 3,00%. |

Beispiel 35

Gemäß einer Arbeitsweise von Beispiel 34 behandelt man β-Diäthylaminoäthylthiol-hydrochlorid mit 10,11-Dihydro-11-hydroxy-2-methylthiodibenz[b,f]oxepin, was 11-[β-(Diäthylamino)-äthylthio]-10,11-dihydro-2-(methylthio)-dibenz[b,f]oxepin als Öl ergibt. Das Öl wird zwecks Reinigung durch eine

Silikagel/Methylenchloridsäule mit 5% Methanol in Methylenchlorid als Eluiermittel chromatographiert. Das gereinigte Öl wird in sein weißes Oxalatsalz vom Schmelzpunkt 118—120°C überführt.

Analyse:

| | | | |
|---|---|---|---|
| Berechnet für $C_{21}H_{27}NOS_2 \cdot C_2H_2O_4$: | C 59,58%; | H 6,31%; | N 3,02%. |
| Gefunden: | C 59,30%; | H 6,32%; | N 2,93%. |

Beispiel 36

Man behandelt eine Probe von 10,11-Dihydro-11-[$\beta$-(dimethylamino)-äthylthio]-2-(methylthio)-dibenz[b,f]oxepin, der freien Base aus Beispiel 34, gemäß der Arbeitsweise von Beispiel 32, wobei man 11-($\beta$-Bromäthylthio)-10,11-dihydro-2-(methylthio)-dibenz[b,f]oxepin erhält. Man löst das Öl in Hexan und kühlt in einem Trockeneis/Aceton-bad, um das Öl als weißen Feststoff vom Schmelzpunkt 64—66°C kristallisieren zu lassen.

Analyse:

| | | | |
|---|---|---|---|
| Berechnet für $C_{17}H_{17}BrOS_2$: | C 53,54%; | H 4,49%; | Br 20,96%. |
| Gefunden: | C 53,72%; | H 4,47%; | Br 21,04%. |

Beispiel 37

Man versetzt ein Gemisch von 2,0 g 2-Fluor-11-[$\beta$-(dimethylamino)-äthylthio]-dibenz[b,f]oxepin, der freien Base aus Beispiel 21, und 62 ml Methanol mit 6,1 g Magnesiumspänen. Man rührt 90 Minuten unter ausreichender Kühlung, um bei der Umsetzung Raumtemperatur aufrechtzuerhalten. Man gießt das methanolische Gemisch ab und kühlt. Das abgekühlte Gemisch wird dann unter vorsichtiger Zugabe mit 60 ml 6 n-Salzsäure behandelt. Man läßt das angesäuerte Gemisch sich auf Raumtemperatur einstellen, bevor es mit 60 ml Wasser verdünnt und danach mit Chloroform extrahiert wird. Die vereinigten Chloroformextrakte wäscht man nacheinander mit 80 ml 10%iger Natronlauge, 100 ml Masser und 25 ml gesättigter Kochsalzlösung und trocknet, was ein gelbes Öl liefert. Man extrahiert dieses dreimal mit je 30 ml heißem Pentan und dampft die vereinigten Pentanextrakte ein, wobei ein reineres Öl verbleibt, das man in Äther zur Ausfällung eines Salzes mit ätherischer Oxalsäure behandelt. Das Salz wird mit Äther gründlich gewaschen, wobei 2-Fluor-10,11-dihydro-11-[$\beta$-(dimethylamino)-äthylthio]-dibenz[b,f]oxepin-oxalat, identisch mit dem in Beispiel 31 beschriebenen Produkt, als weißer Feststoff vom Schmelzpunkt 167—169°C verbleibt.

Beispiel 38

Man rührt die Lösung von 1,5 g 11-[$\beta$-(Brom)-äthylthio]-10,11-dihydro-2-(methylthio)-dibenz[b,f]-oxepin, Beispiel 36, und 0,9 g Kaliumjodid in 15 ml Dimethylformamid bei Raumtemperatur, während man 5 Minuten lang Methylamin in die Lösung einperlt. Nach beendeter Zugabe läßt man 16 Stunden rühren, bevor man nacheinander in 100 ml Eiswasser gießt, mit Benzol extrahiert und die vereinigten Benzolextrakte trocknet. Die getrocknete Lösung wird filtriert und dann zur Trockne eingedampft, wobei ein gelbes Öl verbleibt, das man in sein Maleinsäuresalz, ein körniges Pulver, überführt und aus Methanol/Äther umkristallisiert, was 10,11-Dihydro-11-[$\beta$-(methylamino)-äthylthio]-2-methylthiodibenz[b,f]oxepin-maleinat als Produkt vom Schmelzpunkt 138—140°C liefert.

Analyse:

| | | | |
|---|---|---|---|
| Berechnet für $C_{18}H_{21}NOS_2 \cdot C_4H_4O_4$: | C 59,04%; | H 5,63%; | N 3,13%. |
| Gefunden: | C 58,83%; | H 5,43%; | N 2,90%. |

Beispiel 39

Man perlt Methylamin 20 Minuten lang in die Lösung von 2,6 g 10-($\beta$-Bromäthylthio)-10,11-dihydrodibenz[b,f]oxepin, Beispiel 3, in 15 ml Dimethylformamid. Nach beendeter Zugabe läßt man 16 Stunden bei Raumtemperatur stehen. Danach wird Eiswasser zugegeben und das zweiphasige Gemisch

dreimal mit Äther extrahiert, und die vereinigten Ätherextrakte werden mit Wasser gewaschen. Man trocknet die ätherische Lösung und überführt das so erhaltene Produkt in sein Maleinsäuresalz. Das Salz wird aus Aceton/Äther umkristallisiert, was 10,11-Dihydro-10-[(β-methylamino)-äthylthio]-dibenz[b,f]oxepin-maleinat als Prismen vom Schmelzpunkt 102—104° C liefert.

Analyse:

| | | | | |
|---|---|---|---|---|
| Berechnet für $C_{17}H_{19}NOS \cdot C_4H_4O_4$: | C 62,82%; | H 5,77%; | N 3,49%; | S 7,99%. |
| Gefunden: | C 62,80%; | H 5,77%; | N 3,36%; | S 8,18%. |

Beispiel 40

Man rührt ein Gemisch aus 2-Chlor-10,11-dihydro-11-[β-(dimethylamino)-äthylthio]-dibenz[b,f]oxepin, der freien Base aus Beispiel 6, 0,7 g Bromcyan und 0,8 g Kaliumcarbonat in 20 ml Methylenchlorid 4 Stunden bei Raumtemperatur. Danach wird das Gemisch filtriert und das Filtrat im Vakuum eingeengt, wobei ein viskoses Öl verbleibt. Man chromatographiert dieses über Silikagel, wobei 11-(β-Bromäthylthio)-2-chlor-10,11-dihydrodibenz[b,f]oxepin als blaßgelbliches Öl vom Rf 0,8 verbleibt.

Analyse:

| | | | |
|---|---|---|---|
| Berechnet für $C_{16}H_{14}BrClOS$: | C 51,98%; | H 3,82%; | S 8,67%. |
| Gefunden: | C 52,26%; | H 3,76%; | S 8,61%. |

Beispiel 41

Man perlt Methylamin 10 Minuten lang in die Lösung von 1,5 g 11-[β-(Brom)-äthylthio]-2-chlor-10,11-dihydrodibenz[b,f]oxepin, Beispiel 40, in 20 ml Dimethylformamid. Danach läßt man die Lösung 16 Stunden bei Raumtemperatur stehen. Diese wird dann zur Trockne eingedampft, wobei ein gelblicher öliger Rückstand verbleibt, der mit Natriumbicarbonat und Äther ins Gleichgewicht gebracht wird. Man sammelt die ätherische Phase und trocknet dann vor dem Einengen im Vakuum, wobei ein klares Öl verbleibt, welches man in ein kristallines Maleinsäuresalz überführt. Das Salz wird aus Aceton/Äther umkristallisiert, was 2-Chlor-10,11-dihydro-11-[β-(methylamino)-äthylthio]-dibenz[b,f]oxepin-maleinat als weiße Prismen vom Schmelzpunkt 138—140° C liefert.

Analyse:

| | | | | |
|---|---|---|---|---|
| Berechnet für $C_{17}H_{18}ClNOS \cdot C_4H_4O_4$: | C 57,86%; | H 5,09%; | N 3,21%; | Cl 8,13%. |
| Gefunden: | C 57,62%; | H 5,00%; | N 3,05%; | Cl 8,33%. |

Beispiel 42

a) Man rührt die Lösung von 28,4 g 2-(4-Methylsulfonylphenoxy)-benzylnitril, 99 ml 95%igem Äthanol, 15,2 g 85%iger Kalilauge und 25 ml Wasser 24 Stunden bei 115° C. Anschließend wird das Reaktionsgemisch zu einem Öl eingeengt. Das Öl lößt man in Wasser, wäscht die wäßrige Lösung mit Äther und säuert mit verdünnter Salzsäure an, was ein Öl ergibt. Dieses Öl wird in Methylenchlorid gelöst und die Lösung nacheinander getrocknet, filtriert und zur Trockne eingeengt, wobei ein hellgelber Feststoff verbleibt. Man chromatographiert den Feststoff durch eine Silikagel/Äthersäule mit 10% Methanol in Äther als Eluiermittel, was als Produkt 2-(4-Methylsulfonylphenoxy)-phenylessigsäure vom Schmelzpunkt 125—127° C liefert.

b) Man rührt ein Gemisch aus 1,0 g 2-(4-Methylsulfonylphenoxy)-phenylessigsäure und 10 ml Polyphosphorsäure 2 Stunden unter Stickstoff bei 90—100° C. Man läßt abkühlen und gießt dann auf 100 ml Eis/Wasserbrei. Die wäßrige Lösung wird mit 20%iger Natronlauge alkalisch gemacht, bevor man sie mit Methylenchlorid extrahiert. Die vereinigten Extrakte werden getrocknet und dann zur Trockne eingedampft, wobei ein Öl verbleibt. Man chromatographiert das Öl durch eine Silikagel/Methylenchloridsäule mit 2% Methanol in Methylenchlorid. Die chromatographierte Lösung wird zur Trockne eingedampft, wobei ein Öl verbleibt, das beim Stehen erstarrt. Der Feststoff wird mit Pentan angerieben, was 10,11-Dihydro-2-methylsulfonyl-11-oxodibenz[b,f]oxepin vom Schmelzpunkt 105—106° C liefert.

c) Man behandelt die Lösung von 4,4 g 10,11-Dihydro-2-methylsulfonyl-11-oxodibenz[b,f]oxepin, 4,3 g Dimethylaminoäthylthiol-hydrochlorid und 37 ml Eisessig unter Rühren mit 15 ml Bortrifluoridätherat. Danach gießt man in 300 ml kalte 10%ige Natronlauge und extrahiert die so erhaltene Lösung mit Methylenchlorid. Die vereinigten Extrakte werden mit Wasser gewaschen und vor dem Filtrieren getrocknet. Das Filtrat wird zur Trockne eingedampft, wobei ein Öl verbleibt, welches man über Silikagel/Methylenchlorid mit 2—4% Methanol in Methylenchlorid als Eluiermittel chromatographiert. Man dampft die chromatographierte Lösung zur Trockne ein und überführt das so erhaltene Öl in das weiße Maleinsäuresalz, Schmelzpunkt 137—139° C, von 11-[$\beta$-Dimethylamino)-äthylthio]-2-methylsulfonyldibenz[b,f]oxepin.

Analyse:

| | | | |
|---|---|---|---|
| Berechnet für $C_{19}H_{21}NO_3S_2 \cdot C_4H_4O_4$: | C 56,19%; | H 5,13%; | N 2,85%. |
| Gefunden: | C 56,24%; | H 5,15%; | N 2,86%. |

**Patentansprüche**

1. Verbindungen der Formel

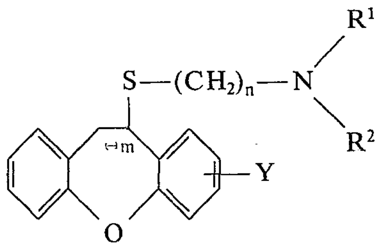

(I)

worin Y für Wasserstoff, Halogen, $C_{1-4}$-Alkyl, $C_{1-6}$-Alkylthio, $C_{1-4}$-Alkylsulfonyl steht, $R^1$ Wasserstoff, geradkettiges oder verzweigtes $C_{1-4}$-Alkyl, Cycloalkyl-$C_{1-3}$-alkyl, wobei der Cycloalkylring 3 bis 5 Wasserstoffatome aufweist, oder Propargyl und $R^2$ geradkettiges oder verzweigtes $C_{1-4}$-Alkyl bedeutet, oder $R^1$ und $R^2$ zusammen mit dem Stickstoffatom, an das sie gebunden sind, einen Heterocyclus bilden, welcher Morpholino, Piperazinyl oder N-substituiertes Piperazinyl mit $C_{1-3}$-Alkyl als N-Substituent darstellt, m die Zahl 0 oder 1 bedeutet, und n für eine ganze Zahl von 2 bis 4 steht, sowie deren physiologisch unbedenkliche Säureadditionssalze.

2. 2-Fluor-11-[$\beta$-methylamino)-äthylthio]-dibenz[b,f]-oxepin, sowie dessen physiologisch unbedenkliche Säureadditionssalze.

3. 10-[$\beta$-(Methylamino)-äthylthio]-dibenz[b,f]-oxepin, sowie dessen physiologisch unbedenkliche Säureadditionssalze.

4. 10,11-Dihydro-10-[($\beta$-dimethylamino)-äthylthio]-dibenz[b,f]oxepin, sowie dessen physiologisch unbedenkliche Säureadditionssalze.

5. 2-Fluor-10,11-dihydro-11-[$\beta$-(dimethylamino)-äthylthio]-dibenz[b,f]oxepin, sowie dessen physiologisch unbedenkliche Säureadditionssalze.

6. Eine Verbindung der Formel I gemäß Anspruch 1 zur Verwendung als Arzneimittel.

7. Verbindungen der Formel

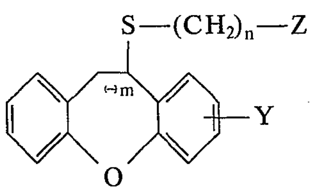

(IV)

worin Y für Wasserstoff, Halogen, $C_{1-4}$-Alkyl, $C_{1-6}$-Alkylthio oder $C_{1-4}$-Alkylsulfonyl steht, Z für Halogen oder

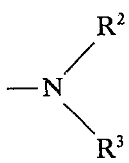

steht, wobei $R^2$ $C_{1-4}$-Alkyl und $R^3$ $C_{1-6}$-Alkoxycarbonyl oder Phenoxycarbonyl bedeutet, m die Zahl 0 oder 1 bedeutet und n für eine ganze Zahl von 2 bis 4 steht, zur Herstellung von Verbindungen nach Anspruch 1.

8. Verfahren zur Herstellung von Verbindungen der Formel I gemäß Anspruch 1, dadurch gekennzeichnet, daß man eine Verbindung der Formel

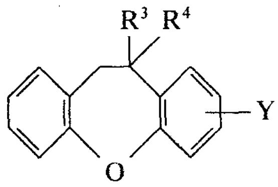

(II)

worin Y die in Anspruch 1 angegebene Bedeutung hat sowie $R^3$ und $R^4$ verschieden sind und jeweils für Wasserstoff oder Hydroxyl oder $R^3$ und $R^4$ zusammen für Sauerstoff stehen, mit einem Aminoalkylthiol der Formel

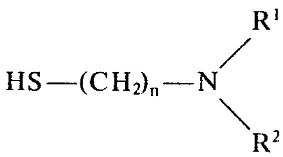

(III)

worin $R^1$ und $R^2$ gleich oder verschieden sind und jeweils geradkettiges oder verzweigtes $C_{1-4}$-Alkyl bedeuten und n die in Anspruch 1 genannte Bedeutung hat, zu einer Verbindung der Formel

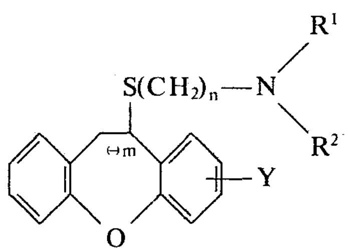

(I)

umsetzt, und gegebenenfalls die erhaltene Verbindung der Formel I, worin m die Zahl 1 darstellt, zu einer Verbindung der Formel I, worin m = 0 ist, reduziert.

9. Verfahren zur Herstellung von Verbindungen der Formel I gemäß Anspruch 1, dadurch gekennzeichnet, daß man eine Verbindung der Formel IVa

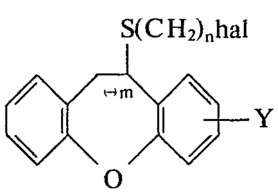

(IVa)

worin Y, m und n die zur Formel I in Anspruch 1 angegebenen Bedeutungen haben, nach üblichen Methoden mit einem entsprechenden acyclischen oder cyclischen Amin umsetzt zu einer Verbindung der Formel I

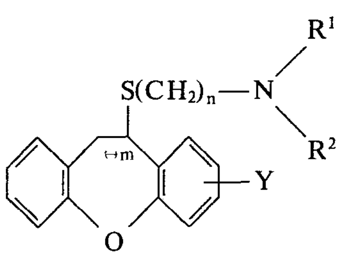

(I)

worin Y, $R^1$, $R^2$, m und n die in Anspruch 1 angegebenen Bedeutungen haben.

10. Verfahren zur Herstellung von Verbindungen der Formel I gemäß Anspruch 1, dadurch gekennzeichnet, daß man eine Verbindung der Formel IVb

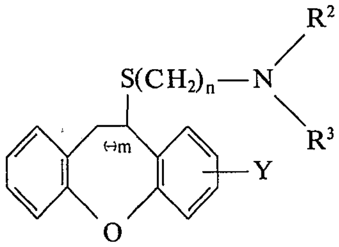

(IVb)

worin Y, m und n die zur Formel I in Anspruch 1 angegebene Bedeutung haben, $R^2$ $C_{1-4}$-Alkyl darstellt und $R^3$ $C_{1-6}$-Alkoxycarbonyl oder Phenoxycarbonyl bedeutet, mit einer Base zu einer Verbindung der Formel I, worin $R^1$ Wasserstoff, $R^2$ geradkettiges oder verzweigtes $C_{1-4}$-Alkyl bedeuten und Y, m und n die im Anspruch angegebene Bedeutung haben, und gegebenenfalls die erhaltene Verbindung der Formel I mit einem $C_{1-4}$-Alkylhalogenid, Propargylhalogenid oder einem $C_{3-5}$-Cycloalkyl-$C_{1-3}$-alkylhalogenid umsetzt zu einer Verbindung der Formel I, worin Y, $R^2$, m und n die in Anspruch 1 angegebene Bedeutung haben und $R^1$ $C_{1-4}$-Alkyl, Propargyl, oder $C_{3-5}$-Cycloalkyl-$C_{1-3}$-alkyl bedeutet.

## Claims

1. Compounds of the formula

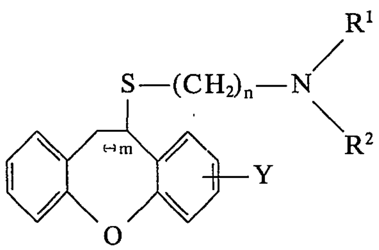

(I)

wherein Y represents hydrogen, halogen, $C_{1-4}$alkyl, $C_{1-6}$-alkylthio, $C_{1-4}$alkylsulfonyl, $R^1$ is hydrogen, straight or branched chain $C_{1-4}$alkyl, cycloalkyl-$C_{1-3}$alkyl wherein the cycloalkyl ring contains from 3 to 5 carbon atoms, or propargyl, $R^2$ is straight or branched chain $C_{1-6}$alkyl or $R^1$ and $R^2$ taken togheter with the nitrogen atom to which they are attached form a heterocycle which is morpholino, piperazinyl or N-substituted piperazinyl in which the N-substituent is $C_{1-3}$alkyl; m is the integer 0 or 1; and n is an integer of from 2 to 4; and physiologically tolerable acid addition salts thereof.

2. 2-Fluoro-11-[β-(methylamino)ethylthio]dibenz[b,f]-oxepin and a physiologically tolarable acid addition salt thereof.

3. 10-[β-(Methylamino)ethylthio]-dibenz[b,f]oxepin and a physiologically tolerable acid addition salt thereof.

4. 10,11-Dihydro-10-[(β-dimethylamino)ethylthio]dibenz[b,f]oxepin and a physiologically tolerable acid addition salt thereof.

5. 2-Fluoro-10,11-dihydro-11-[β-(dimethylamino)ethylthio]dibenz[b,f]oxepin and a physiologically tolerable acid addition salt thereof.

6. A compound of the formula I as claimed in claim 1 suitable for the use as medicament.

7. Compounds of the formula

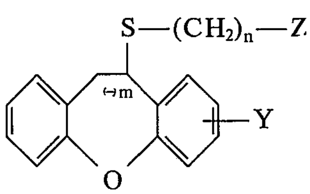

(IV)

wherein Y can be hydrogen, halogen, $C_{1-4}$alkyl, $C_{1-6}$alkylthio or $C_{1-4}$alkylsulfonyl; Z is halogen or

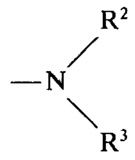

with $R^2$ being $C_{1-4}$alkyl and $R^3$ being $C_{1-6}$alkoxycarbonyl or phenoxycarbonyl; m is zero or 1 and n is an integer of from 2 to 4, for the preparation of compounds as defined in claim 1.

8. A process for the preparation of compounds of the formula I as defined in claim 1, which comprises reacting a compound of the formula

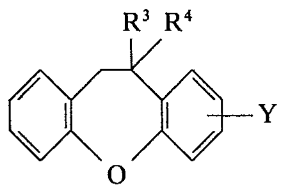

(II)

wherein Y is as defined in claim 1 and $R^3$ and $R^4$ are different and each is hydrogen or hydroxy or $R^3$ and $R^4$ together are oxygen, with an aminoalkylthiol of the formula

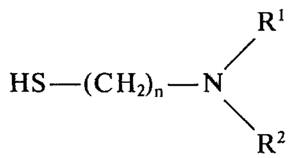

(III)

wherein $R^1$ and $R^2$ are the same or different and each can be straight-chain or branched $C_{1-4}$alkyl, and n is as defined in claim 1 to produce a compound of the formula

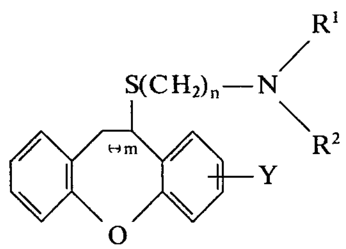

(I)

and optionally reducing the compound of the formula I obtained, wherein m is the integer 1, to obtain a compound of the formula I wherein m is the integer 0.

9. The process for the preparation of compounds of the formula I as defined in claim 1, which comprises reacting a compound of the formula IVa

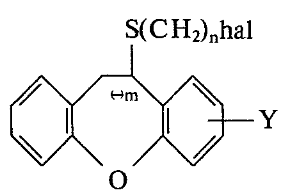

(IVa)

wherein Y, m and n are defined as in formula I in claim 1, by usual methods with a correspondig acyclic or cyclic amine, to produce a compound of the formula I

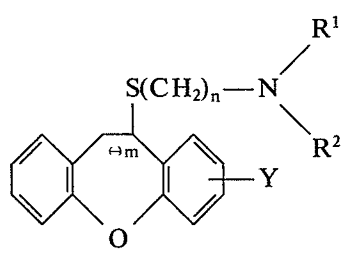

(I)

wherein Y, $R^1$, $R^2$, m and n are defined as in claim 1.

10. The process for the preparation of compounds of the formula I as defined in claim 1, which comprises reacting a compound of the formula IVb

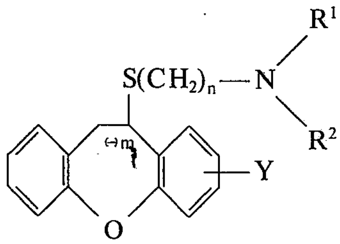

(IVb)

wherein Y, m and n are defined as in formula I in claim 1; $R^2$ is $C_{1-4}$alkyl and $R^3$ is $C_{1-6}$alkoxycarbonyl or phenoxycarbonyl, with a base, to produce a compound of the formula I wherein $R^1$ is hydrogen, $R^2$ is straight-chain or branched $C_{1-4}$alkyl and Y, m and n are defined as in claim 1, and optionally reacting the compound of the formula I obtained with a $C_{1-4}$alkyl halide, propargyl halide or a $C_{3-5}$cycloalkyl-$C_{1-3}$alkyl halide to produce a compound of the formula I wherein Y, $R^2$ m and n are defined as in claim 1 and $R^1$ is $C_{1-4}$alkyl, propargyl or $C_{3-5}$cycloalkyl-$C_{1-3}$alkyl.

**Revendications**

1. Composés de formule

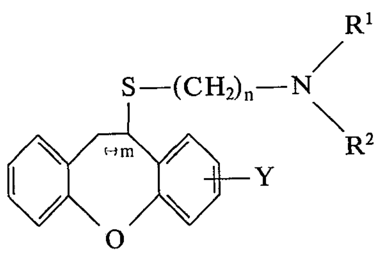

(I)

dans laquelle Y représente l'hydrogene, un halogène ou un groupe alkyle en $C_1-C_4$, alkylthio en $C_1-C_6$ ou alkylsulfonyle en $C_1-C_4$, $R^1$ représente l'hydrogène, un groupe alkyle en $C_1-C_4$ à chaîne droite ou ramifiée, un groupe cycloalkyl-($C_1-C_3$) alkyle dans lequel le noyau cycloalkyle a de 3 à 5 atomes de carbone, ou un groupe propargyle et $R^2$ représente un groupe alkyle en $C_1-C_4$ à chaîne droite ou ramifiée, ou bien $R^1$ et $R^2$ forment ensemble avec l'atome d'azote sur lequel ils sont fixés, un hétérocycle morpholino, pipérazinyle ou pipérazinyle N-substitué avec un groupe alkyle en $C_1-C_3$ comme substituant sur l'azote, m est 0 ou 1 et n est un nombre entier de 2 à 4; et leurs sels d'addition avec des acides physiologiquement acceptables.

2. 2-fluoro-11-[β-méthylamino)-éthylthio]-dibenz[b,f]-oxépine et ses sels d'addition avec des acides physiologiquement acceptables.

3. 10-[β-(méthylamino)-éthylthio]-dibenz[b,f]oxépine et ses sels d'addition avec des acides physiologiquement acceptables.

4. 10,11-dihydro-10-[(β-diméthylamino)-éthylthio]-dibenz[b,f]-oxépine et ses sels d'addition avec des acides physiologiquement acceptables.

5. 2-fluoro-10,11-dihydro-11-[β-(diméthylamino)-éthylthio]-dibenz[b,f]oxépine et sels d'addition avec des acides physiologiquement acceptables.

6. Composé de formule I selon la revendication 1, pour utilisation comme médicament.

7. Composés de formule

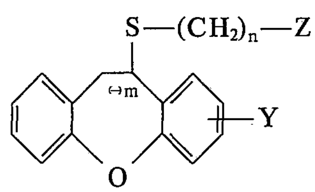

(IV)

dans laquelle Y représente l'hydrogène, un halogène ou un groupe alkyle en $C_1-c_4$, alkylthio en $C_1-C_6$ ou alkylsulfonyle en $C_1-C_4$, Z représente un halogène ou

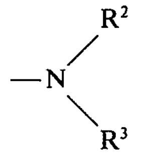

$R^2$ représentant un groupe alkyle en $C_1-C_4$ et $R^3$ un groupe alcoxycarbonyle en $C_1-C_6$ ou phénoxycarbonyle, m est 0 ou 1 et n est un nombre entier de 2 à 4, pour la préparation de composés selon la revendication 1.

8 – Procédé de préparation de composés de formule I selon la revendication 1, caractérisé en ce qu'on fait réagir un composé de formule

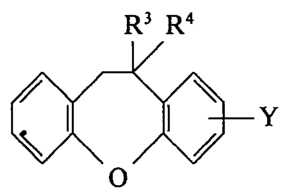

(II)

dans laquelle Y a la signification indiquée dans la revendication 1 et $R^3$ et $R^4$ sont différents et représentent chacun l'hydrogène ou un groupe hydroxyle ou bien $R^3$ et $R^4$ représentent ensemble l'oxygène, avec un composé aminoalkylthio de formule

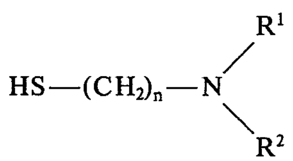

(III)

dans laquelle $R^1$ et $R^2$ sont identiques ou différents et représentent chacun un groupe alkyle en $C_1-c_4$ à chaîne droite ou ramifiée et n a la signification indiquée dans la revendication pour obtenir un composé de formule

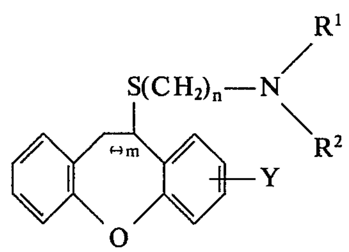

(I)

et éventuellement on réduit le composé de formule I obtenu, dans lequel m est égal à 1, en un composé de formule I dans lequel m = 0.

9 – Procédé de préparation de composés de formule I selon la revendication 1, caractérisé en ce qu'on fait réagir un composé de formule IVa

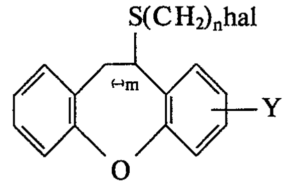

(IVa)

dans laquelle Y, m et n ont les significations indiquées pour la formule I dans la revendication 1, selon des procédés usuels, avec une amine acyclique correspondante pour obtenir un composé de formule I

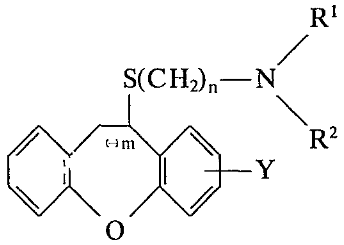

(I)

dans laquelle Y, $R^1$, $R^2$, m et n ont les significations indiquées dans la revendication 1.

10 – Procédé de préparation de composés de formule I selon la revendication 1, caractérisé en ce qu'un fait réagir un composé de formule IVb

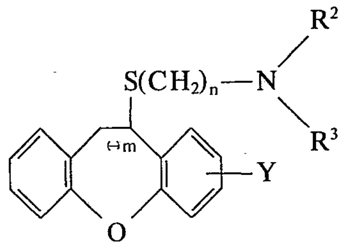

(IVb)

dans laquelle Y, m et n ont les significations indiquées pour la formule I dans la revendication 1, $R^2$ représente un groupe alkyle en $C_1-C_4$ et $R^3$ un groupe alcoxycarbonyle en $C_1-C_6$ ou phénoxycarbonyle, avec une base, pour obtenir un composé de formule I dans lequel $R^1$ représente l'hydrogène, $R^2$ représente un groupe alkyle en $C_1-C_4$ à chaîne droite ou ramifiée et Y, m et n ont les significations indiquées dans la revendication 1; et éventuellement on fait réagir le composé de formule I obtenu avec un halogénure d'alkyle en $C_1-C_4$, un halogénure de propargyle ou un halogénure de cycloalkyl $(C_1-C_3)$-alkyle dans lequel le noyau cycloalkyle a de 3 à 5 atomes de carbone, pour obtenir un composé de formule I dans lequel Y, $R^2$, m et n ont les significations indiquées dans la revendication 1 et $R^1$ représente un groupe alkyle en $C_1-C_4$, propargyle ou cycloalkyl-$(C_1-C_3)$-alkyle dans lequel le noyau cycloalkyle a de 3 à 5 atomes de carbone.